# EUROPEAN PATENT APPLICATION

(11) **EP 2 896 386 A1**
(43) Date of publication of application: **22.07.2015**
(21) Application number: 15150999.9
(22) Date of filing: 27.01.2006
(51) Int. Cl.: A61F 2/14

(54) **DEFORMABLE CORNEAL IMPLANTS**

(30) Priority: 31.01.2005 US 648949 P
(62) Divisional of application: 06719673.3
(71) Applicant: Shiuey, Yichieh, San Jose, CA 95129 (US)
(72) Inventor: Shiuey, Yichieh, San Jose, CA 95129 (US)
(74) Representative: Clark, Jane Anne

(57) **Abstract**

A corneal implant (15), wherein the corneal implant is reversibly deformable, the deformed implant being capable of implantation in a corneal pocket through a corneal incision which is smaller than the width of the undeformed implant.

## Description

### BACKGROUND OF THE INVENTION

1. Field of the Invention. There are many different types of corneal implants that have been developed for the treatment of refractive error and disease. Because of limitations in the methods of creating corneal pockets, these implants have all been designed for placement in the cornea by creation of a corneal incision which is either similar in size to the smallest dimension of the implant or larger. Recently, two methods of corneal pocket creation have been devised which can create a pocket with an external opening width that is less than the maximum internal width of the pocket. These two methods are pocket creation by the femtosecond laser and, of particular interest, cornea cutting, as described in US 2004/0243159 and 0243160, invented by the inventor herein, the full disclosure of which is incorporated herein by reference.

It is advantageous to have a biocompatible corneal implant that can be placed through an external incision that is less than the width of the implant, especially an external incision that is less than half of the width of the implant. A small external incision decreases induced surgical astigmatism and speeds up the recovery time for the patient. Moreover, it is useful to have a relatively large implant that can be placed through a relatively small incision. For example a lens implant that is larger is more likely to give good quality vision especially in a patient with large pupils. It is also advantageous to have a simple and reliable delivery system for the corneal implant.

2. Description of the Background Art. Intraocular lenses for cataract surgery have been designed to be placed through a small incision. These small incision cataract surgery lenses cannot practically be used within a corneal pocket. Most small incision cataract surgery lens implants are usually too thick to be placed within a corneal pocket. For example the typical thickness of a cataract surgery lens implant is 1 nun or more which is substantially thicker than the human cornea, which is usually between 0.5 to 0.6 mm. Some corneal implants that have been designed only have a thickness of about 0.05 mm. Moreover, the cataract surgery lens implants have haptics, which are extensions from the lens implant designed to keep the lens implant fixated within the capsular bag. Haptics are not present and not necessary for corneal implants. Finally, the cataract surgery lens implants are not designed to be biocompatible with the cornea and would not be tolerated as corneal implants.

The delivery systems designed for small incision cataract surgery lens implants are not well adapted for use as a delivery system for small incision corneal implants. These delivery systems have been designed for cataract surgery lens implants that are much thicker than the usual corneal implant. The delivery systems for small incision cataract surgery lens implants are designed to accommodate haptics, which would not be present on a corneal lens implant.

### BRIEF SUMMARY OF THE INVENTION

Improved corneal implants and systems and methods for implanting corneal implants are provided by the present invention. These corneal implants can be placed through a corneal incision that is substantially less than the width of the implant. In preferred aspects, the corneal incision is equal to or less than half of the width of the implant.

In accordance with a first aspect of the present invention, the corneal implant is reversibly deformable in shape to allow its passage through a corneal incision that is equal or less than half of the width of the implant. The corneal implant is bio-compatible with the cornea, the eye, and the body. Any material which can meet these criteria may potentially be used for the implant. Possible materials include one or more compounds selected from the group consisting of collagen, polyurethanes, poly(2-hydroxyethylmethacrylate), polyvinylpyrolidone, polyglycerolmethacrylate, polyvinyl alcohol, polyethylene glycol, polymethacrylic acid, silicones, acrylics, polyfluorocarbons, and polymers with phosphocholine. In a preferred embodiment, the material comprises a hydrogel. In additional preferred embodiments, the material comprises polymethacrylic acid-co hydroxyethyl methacrylate (PHEMA/MAA). In other preferred embodiments, holes or pores may be provided in the implant to increase biocompatibility of the implant by allowing nutritive substances and gasses (e.g., water, glucose, and oxygen) to pass easily through the implant in order to maintain healthy metabolism in the cornea. In still other preferred embodiments, the polymer material may have thermoplastic properties such that the implant will have one desired shape at one temperature and then deform into another desired shape at a second temperature. In yet other preferred aspects, the corneal implant may comprise one or more separate, smaller components that can be assembled *-in situ* placed inside the corneal pocket. Such *in situ* assembly advantageously minimizes the incision size needed to insert a corneal implant.

The corneal implant may be of any shape that allows it to be placed within a corneal pocket. In preferred embodiments, the corneal implant is substantially round. In alternate preferred embodiments, the corneal implant is not round. A corneal implant which is not round has the advantage that it is less likely to rotate within a corneal pocket. This property is useful in the implants which correct for astigmatism.

In preferred other embodiments, the corneal implant is a lens. The lens can be a monofocal, multifocal, Fresnel, diffractive, prismatic, or other type of lens that can be used to treat refractive error (such as myopia, hyperopia, or astigmatism) presbyopia, or ocular disease e.g. macular degeneration. The lens may also be made of a polymer that can have its refractive properties adjusted permanently or reversibly by electromagnetic energy as described in U.S. Patent Application 2003/0173691 to Jethmalani.

The corneal implant usually comprises a prosthesis that is used to replace or augment a portion of the cornea. Such implants are useful in restoring optical clarity or structural integrity to the cornea in lieu of corneal transplantation. The corneal prosthesis may be used to replace only a partial thickness portion of the cornea or a full thickness portion of the cornea. In preferred aspects, the corneal implant may be coated with extracellular matrix proteins such as collagen, fibronectin, laminin, substance P, insulin-like growth factor-1, or peptide sequences such as fibronectin adhesion-promoting peptide (FAP). In additional preferred aspects, these extracellular matrix proteins and peptides are tethered or otherwise bound to the epithelial side of the corneal implant by the methods described in U.S. Patent 6,689,165, to Jacob et al. Such surface treatments are intended to promote epithelialization on the surface of a corneal implant.

In alternate preferred embodiments, the surface of the corneal implant may have a texture that promotes epithelialization on the surface of the corneal implant. Textures, such as surface indentations, may be applied to the surface of the corneal implant to promote epithelialization, as described in U.S. Patent 6,454,800 to Dalton et al.

In yet other alternate preferred embodiments, the corneal implant may be manufactured from a material that promotes epithelialization on the surface of the corneal implant. Examples of such materials include polymers selected from the group consisting of collagen and N-isopropylacrylamide, collagen and 1-ethyl-3.3'(dimethyl-aminopropyl)-carbodiimide as well as collagen and N-hydroxysuccinimide (EDC/NHS). In further preferred aspects, the polymer may additionally contain extracellular matrix proteins such as fibronectin, laminin, substance P, insulin-like growth factor-1, or peptide sequences such as fibronectin adhesion-promoting or peptide (FAP)

Optionally, at least a portion of the device may contain holes or be porous in nature so as to promote growth of corneal tissue into and through the implant in order to promote retention and biocompatibility. Such porous implants may be fabricated as described in U.S. Patent 6,976,997 to Noolandi et al. and U.S. Patent 5,300,116 to Chirila et al.

Optionally, at least a portion of the lens or other corneal implant may be colored. Coloration can be useful for cosmetic purposes or for therapeutic purposes e.g. treatment of aniridia. For example, methods of applying biocompatible inks, which are well known in colored contact lens manufacturing, may be used to color the corneal implant. Particular coloring methods are described in U.S. Patent Applications 2003/0054109 and 2003/0025873, the disclosures of which are incorporated herein by reference. In alternate preferred aspects, the corneal implant may be colored with photosensitive inks that change color with exposure to electromagnetic waves. This allows the color of the corneal implant to be adjusted permanently or reversibly by exposure to electromagnetic waves *in vivo.*

Optionally, the corneal implant may also contain an ultraviolet filter compound of the benzophenone type such as 3-(2 Benzyotriazolyl)-2-Hydroxy-5-Tert-Octyl-Benzyl Methacryl Amide.

In yet other alternate preferred embodiments, the corneal implant may be a device. Examples of potential implant devices include miniature cameras and aqueous glucose monitors.

The improved corneal implants of the present invention are deformable into a reduced width shape that allows passage through a corneal incision that is substantially less than the width of the implant when undeformed or unconstrained. In preferred aspects, the incision will be less than or equal to one-half of the width of the implant.

A system according to the present invention comprises a hollow member and implant mover or other axial pusher used to deliver a corneal implant that has been constrained to fit inside an axial hollow passage of the hollow member. The implant may be deformed or constrained in any shape or configuration having a "reduced width" that allows it to be fit inside of the hollow member e.g., rolled or folded. By "reduced width" it is meant that a maximum width of the implant, such as a diameter of a circular lens, is reduced by some threshold amount, typically by at least one-half (50%), often by at least 60%, and sometimes by 65% or more.

Once the corneal implant is inside the hollow member, the implant mover or other axial pusher is used to engage and push the implant into the corneal pocket. Optionally, the system may further comprise a deformation chamber where the implant is deformed into a shape and size that will fit inside the hollow member. In other preferred aspects, the deformation chamber may contain ridges, protrusions, indentations, or recesses which help to maintain orientation of the corneal implant within the deformation chamber during the deformation process. Optionally, the hollow member is tapered, i.e., narrower at a distal end than at a proximal end. Such tapering allows additional deformation (size or width reduction) of the implant as it is advanced through the hollow member and passes out through a smaller distal opening. The interior of the hollow member may contain ridges, protrusions, indentations, or recesses which help to maintain orientation of the corneal implant as it travels inside of the hollow member. The system for implant placement is designed to allow an implant to be placed into a corneal pocket with an entry incision that is equal or less than one-half of the width of the implant, however, the system can also be used to place an implant through a corneal incision that is greater than one-half of the width of the implant.

### BRIEF DESCRIPTION OF THE DRAWINGS

Figures 1A, 1B, 1C, and 1D illustrate prior art corneal implants.
Figures 2A through 2C illustrates a first embodiment of apparatus of the present invention.
Figures 3A through 3C illustrate side views of a corneal implant as it is advanced and constrained by the apparatus of Figures 2A-2C.
Figures 4A through 4D illustrate a second embodiment of the apparatus of the present invention.
Figures 5A through 5D illustrate side views of a corneal implant as it is advanced and constrained by the apparatus of Figures 4A-4D.
Figures 6A through 6C illustrate a third embodiment of the apparatus of the present invention.
Figures 7A and 7B illustrate use of the apparatus of Figures 6A-6C in implanting an implant in a cornea.
Figures 8A through 8D illustrate preferred corneal implants in accordance with the present invention.
Figures 9A through 9F illustrate a further implantation protocol in accordance with the present invention.
Figures 10A through 10F illustrate a further implantation protocol in accordance with the present inventions.
Figures 11A through 11F illustrate a further implantation protocol in accordance with the present inventions.
Figures 12A and 12B illustrate a tool in accordance with the principles of the present invention for collapsing and advancing a corneal implant.

### DETAILED DESCRIPTION

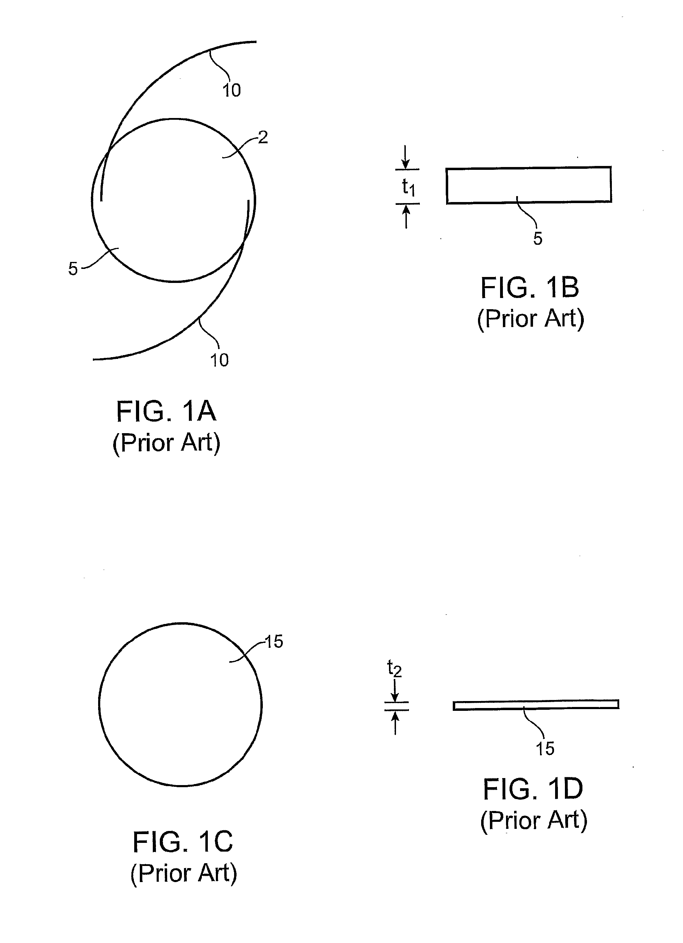

Figure 1A shows a top view of a cataract surgery lens implant 2. A round optic 5 of the implant 2 has haptics 10 which extend from the periphery of the optic. The haptics 10 are used to help the optic center and fixate within the capsular bag. Figure 1B shows a side view of a cataract surgery lens implant optic 5. Note that the thickness t₁ of the optic 5 is typically 1 mm or more and is substantially greater than the 0.5 to 0.6 mm thickness of the human cornea. The thickness of the optic 5 makes it inappropriate for use as a corneal lens implant. Figure 1C shows a top view of a corneal implant 15. Note there are no haptics on the corneal implant. Figure 1D shows a side view of corneal implant 15. Note that the thickness t₂ is substantially less than cataract surgery lens implant 5. The thickness t₂ of corneal implant 15 would in general be less than the thickness of the human cornea.

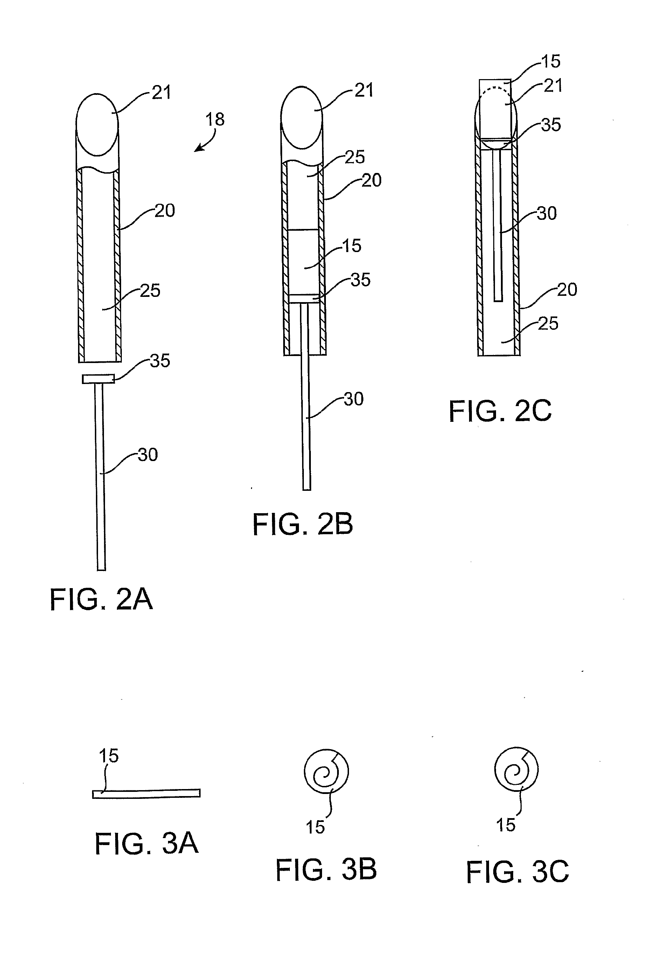

Figure 2A shows a corneal implant delivery system 18 in partial section. A hollow member 20 having a distal tip 21 (which is preferably beveled or chamfered) defines hollow axial passage 25 (e.g. an axial lumen). Axial pusher 30 has a tip 35 that engages a corneal implant 15 that has been deformed in shape and constrained to fit inside the hollow axial passage 25 of the hollow member 20, as shown in Figure 2B. The cross-section of hollow passage 25 may be circular, polygonal, or any other shape that is conducive to constraining the corneal implant 15. The hollow axial passage 25 of the hollow member 20 may contain ridges, protrusions, indentations, or recesses (now shown) which help to maintain orientation of the corneal implant as it advances distally of the hollow member (not shown). Axial pusher 30 engages one end of the constrained corneal implant 15 to advance the constrained implant through hollow passage 25. Figure 2C shows the constrained corneal implant 15 emerging from a distal end of the hollow passage 25 still in its deformed and constrained configuration. By placing the tip of the hollow member 20 through an incision in the cornea, the corneal implant 15 may be advanced into the corneal pocket (not shown) through even a very small incision. In preferred aspects, the corneal implant is able to pass through an entry incision that is less than one-half the width of the corneal implant. In those cases, the hollow member will have an external width from 0.5 mm to 5 mm, preferably from 1 mm to 3 mm and an internal width from 0.3 mm to 4.8 mm, preferably from 0.8 mm to 2.8 mm.

Figure 3A shows a side view of corneal implant 15 in its non-deformed, non-constrained shape. Figure 3B and 3C shows an end on view of the corneal implant 15 as it is moved within the hollow member 20. Note that the corneal implant 15 has been deformed and constrained into a rolled configuration. The rolled configuration will preferably have a diameter in the range from 0.3 mm to 4.8 mm, more preferably from 0.6 mm to 2.6 mm, to fit into the hollow passage 25 of the hollow member 20.

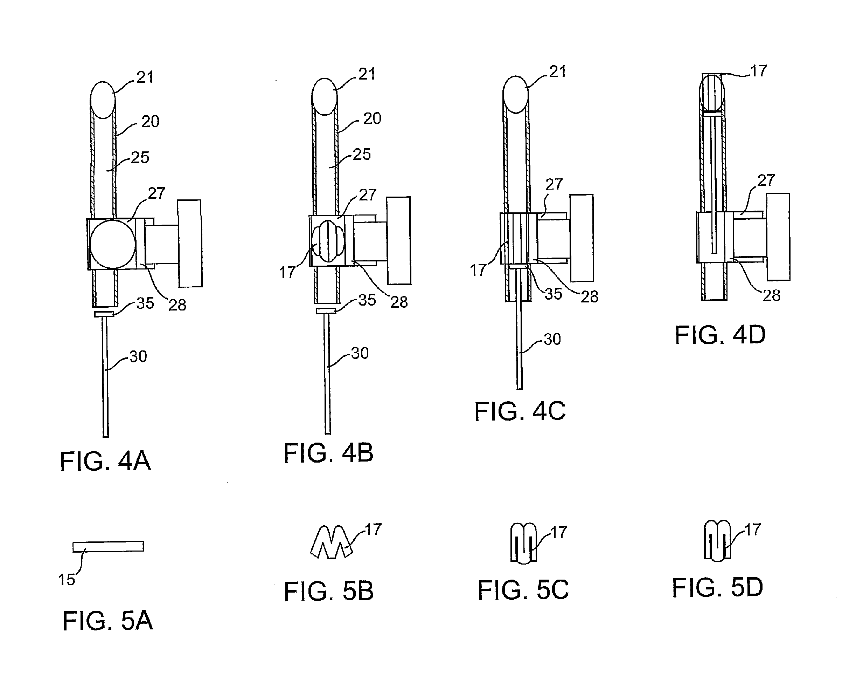

Figure 4A-4D shows a corneal implant delivery system with a deformation chamber 27 and a deforming member 28. In this embodiment of the invention, the corneal implant 15 is placed into the chamber 27 in an unconstrained and undeformed configuration and is then deformed into a folded or rolled corneal implant 17 within deformation chamber 27 by deforming member 28. Deforming member 28 is moved within deformation chamber 27 to deform and fold corneal implant 15 into a folded or rolled corneal implant 17. Figure 4C shows axial pusher 30 engaging deformed corneal implant 17 by implant mover tip 35. Figure 4D shows deformed and folded corneal implant 17. Axial pusher 30 engages corneal implant 17 to push the deformed constrained implant inside hollow passage 25. Figure 4D shows that corneal implant 17 has been advanced by axial pusher 30 out of the hollow passage 25 while retaining a constrained shape. The constrained configuration of corneal implant 17 allows passage into the corneal pocket (not shown) through a small incision. The presence of the optional deformation chamber 27 and deforming member 28, advantageously allows the corneal implant 15 to be easily deformed into a configuration that will allow it to be placed through a small corneal incision into a corneal pocket.

Figures 5A-5D show side views of the corneal implant 15 being deformed into an exemplary deformed and folded or pleated corneal implant 17.

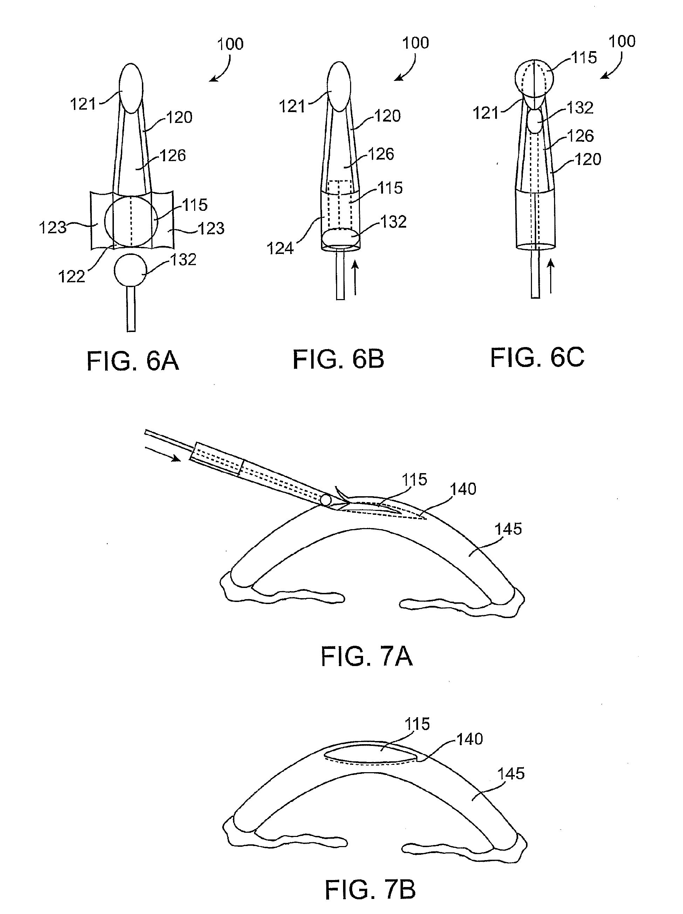

Figures 6A-6C show a top view of an alternative corneal implant delivery system 100. In this embodiment a corneal implant 115 is placed into a deformation area 122. When the "wings" 123 of the deformation area are closed, a deformation chamber 124 (Fig. 6B) is formed which deforms the corneal implant 115. In this embodiment, the corneal implant 115 is folded in half. A tip 132 of an axial pusher 130 engages corneal implant 115. The hollow member 120 is tapered so that hollow passage 126 is narrower at a distal end 121 that inserts into the corneal pocket. This allows the corneal implant 115 to be deformed into an even smaller cross-section as the implant is advanced distally and through the distal end 121. Advantageously in this embodiment, the implant mover tip 132 may also be deformable to fit within the narrowing hollow passage 126.

Figure 7A shows a side cross-sectional view of corneal implant 115 being inserted into corneal pocket 140. Figure 7B shows the final shape of corneal implant 115 after it has been inserted into corneal pocket 140 and unfurled or otherwise expanded back to its unconstrained size within cornea 145.

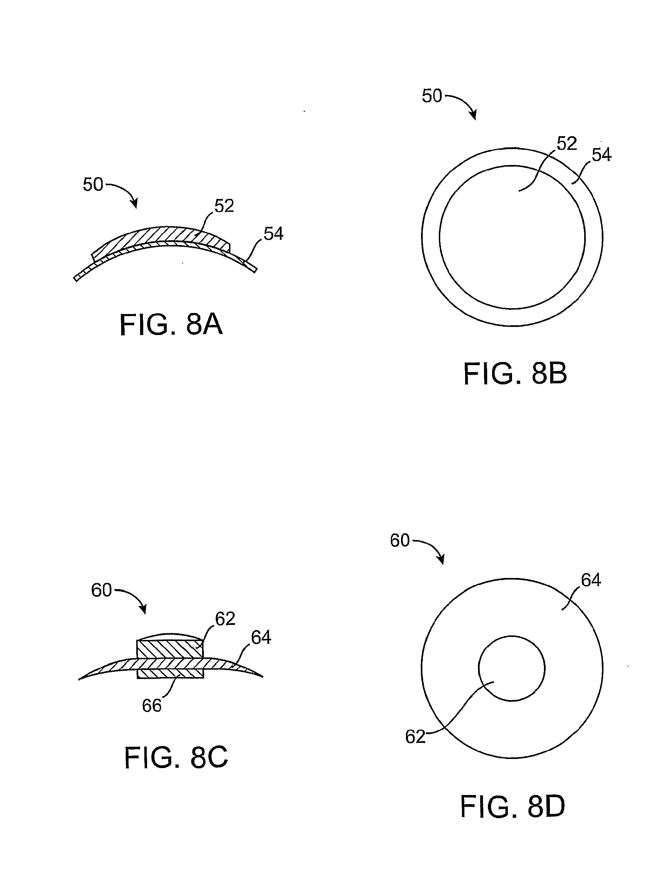

Figure 8A illustrates a cross-sectional view of a corneal implant prosthesis 50 Corneal implant 50 is meant to replace a portion of the anterior layers of the cornea. In this embodiment there is a central optic 52 that protrudes anteriorly from a rim 54. In preferred aspects, the central optic would protrude anteriorly from the rim by 1 to 600 microns. More preferably, the central optic would protrude anteriorly from the rim by 50 to 400 microns. The central optic 52 will replace diseased anterior corneal tissue that has been removed. The rim 54 is designed to partly or fully around the center of optic and to fit within the peripheral recesses of a corneal pocket in order to anchor the corneal implant prosthesis to the cornea. The rim may be a continuous skirt as illustrated or may be crenellated or otherwise distributed in sections about the periphery of the center optic. Figure 8B shows a top view of corneal implant prosthesis 50 which shows the central optic 52 and the rim 54. The rim 54 may optionally contain holes or be porous in nature so as to promote growth of corneal tissue into and through the implant, in order to promote retention and biocompatibility.

Figure 8C shows a cross-sectional view of corneal implant prosthesis 60 which is meant to replace a full-thickness area of the cornea. In this embodiment there is an anterior portion of central optic 62 which protrudes anteriorly from a rim 64. The anterior portion of central optic 62 will replace diseased anterior corneal tissue that has been removed. In preferred aspects, the central optic would protrude anteriorly from the rim by 1 to 600 microns. More preferably, the central optic would protrude anteriorly from the rim by 50 to 400 microns. In addition corneal implant prosthesis 60 has a posterior portion of central optic 66 which protrudes posteriorly from rim 64. In preferred aspects, the central optic would protrude posteriorlyfrom the rim by 1 to 900 microns. More preferably, the central optic would protrude posteriorly from the rim by 50 to 800 microns. The posterior portion of central optic 63 will replace diseased posterior corneal tissue that has been removed. The rim 64 will anchor corneal implant prosthesis 60 within the peripheral recesses of the corneal pocket and provide a water-tight seal. The rim 64 may optionally contain holes or be porous in nature so as to promote growth of corneal tissue into and through the implant, in order to promote retention and biocompatibility. The rim may be formed from any of the lens materials described above.

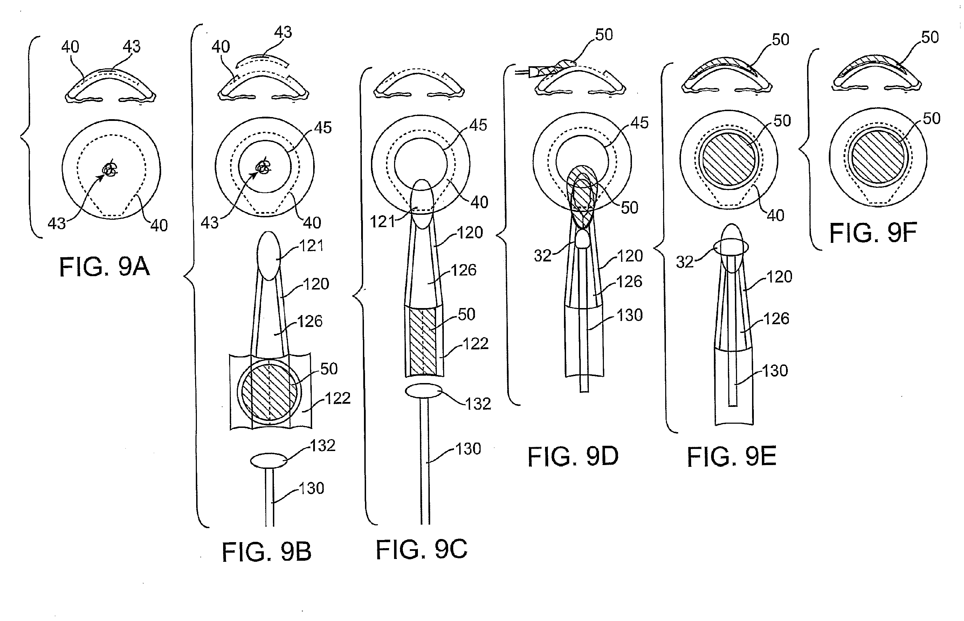

Figures 9A-9F show a method of treating an anterior corneal disease process using the methods and apparatus of the present invention. In each Figure 9A-F, a cross-sectional view of the cornea is seen above and a top view is seen below. In Figure 9A it is shown that pocket 40 has been created posterior to anterior diseased cornea 43. Figure 9B shows that anterior diseased corneal 43 has been excised with a circular trephine (not shown) to create an open top having a peripheral pocket. The edge of the excision is shown as 45. Figure 9B also shows corneal implant 50 resting in the deformation area 122. In Figure 9C the hollow member 120 has been inserted into pocket 40 through external opening 42 and corneal implant 50 has been folded in half within deformation chamber 124. Figure 9D shows that corneal implant 50 has been further deformed into a more compact shape by its movement through narrowing hollow passage 126 and is being extruded into pocket 40. Figure 9E shows that corneal implant 50 has been restored to its original shape within corneal pocket 40. Central optic 52 fills the space left by excised diseased anterior cornea 43 and restores optical clarity to the cornea. Hollow member 120 and implant mover 30 have been withdrawn from corneal pocket 40. Figure 9F shows the final appearance of corneal implant 50 fixated within corneal pocket 40.

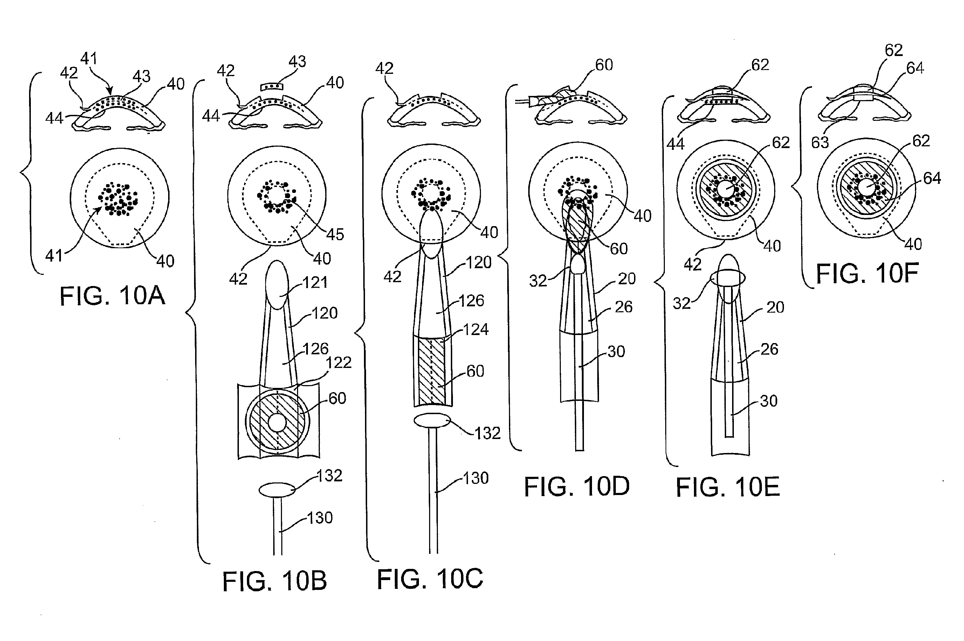

Figures 10A-10F show a method of treating a full-thickness corneal disease (e.g. pseudophakic bullous keratopathy) through the use of the present invention. In each figure10 A-F, a cross-sectional view of the cornea is seen above and a top view is seen below. In figure 10A it is shown that pocket 40 has been created within the layers of the diseased cornea 41. The pocket divides the cornea into diseased anterior cornea 43 and diseased posterior cornea 44. Figure 10B shows that anterior diseased cornea 43 has been excised with a circular trephine (not shown). The edge of the excision is shown in dashed lines as 45. Figure 10B also shows corneal implant 60 resting in the deformation charter or area 122. In Figure 10C the hollow member 120 has been inserted into pocket 40 through external opening 42 and corneal implant 60 has been folded in half within deformation chamber 122. Figure 10D shows that corneal implant 60 has been further deformed into a more compact shape by its movement through narrowing hollow passage 126 and is being extruded into pocket 40. Figure 10E shows that corneal implant 60 has been restored to its original shape within corneal pocket 40. Anterior optic 62 fills the space left by the excised diseased anterior cornea 43. In preferred aspects, after corneal implant 60 has been positioned in the pocket, the posterior diseased cornea 44 can be excised with low profile curved corneal scissors or some other cutting tool (e.g. plasma blade) inserted through external opening 42. Figure 10F shows the final appearance of corneal implant prosthesis 60. Note that the rim 64 anchors corneal implant prosthesis 60 within the peripheral recesses of the corneal pocket and provides a water-tight seal. In this embodiment, posterior optic 63 protrudes through the space left by exicised diseased cornea 44. However, posterior optic 63 is optional and is not necessarily required for the corneal implant to properly function. It is to be understood that the relative dimensions, shapes, and angles of the anterior central optic 62, posterior central optic 63, and rim 64, may each be modified to promote improved retention as well as optical qualities all in keeping within the scope of the present invention.

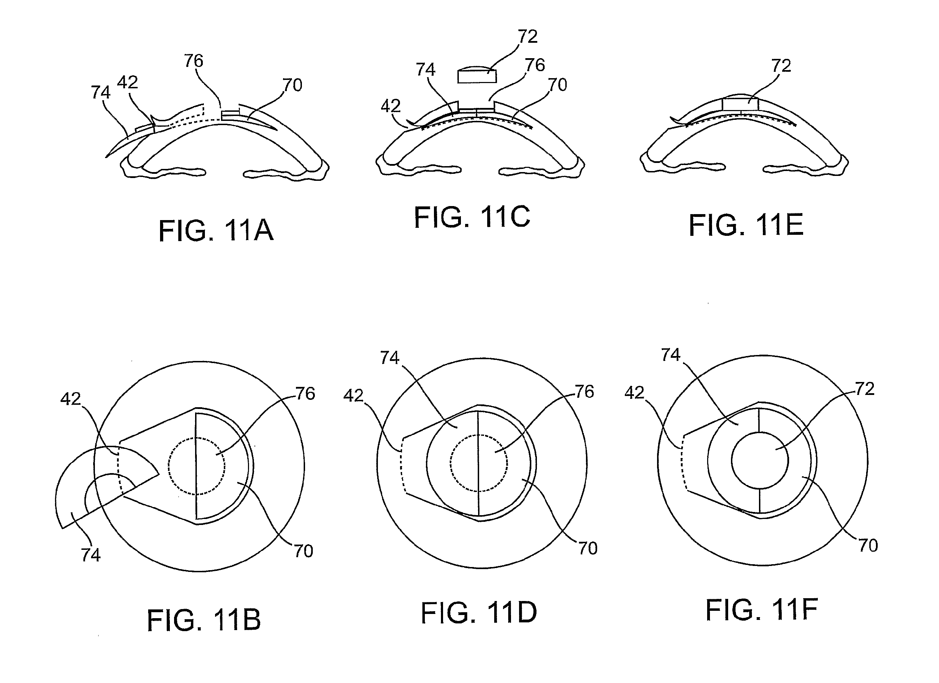

Figure 11A-11F show an embodiment of a corneal implant that can be assembled within the corneal pocket. By assembling individual smaller pieces of the corneal implant within the corneal pocket, a relatively large corneal implant can be constructed while using a relatively small external incision. The top portion of Figures 11A and 11B show a cross-sectional view of a corneal with an intra-stromal pocket. The bottom portion of Figure 11A shows a top down view of a cornea with an intra-stromal pocket. In both Figures 11A and 11B, it can be seen that the first half of the rim 70 has already been inserted inside the pocket. A second half of the rim 74 is being inserted through the small external incision. Note that because the corneal tissue is partially elastic, the rim may be made of a relatively rigid material e.g. polymethylmethacrylate (PMMA) and still be inserted through the external opening 42 that is less than half of the diameter of the assembled corneal implant. The vertical dashed lines in the top of the figure and the circular dashed lines in the bottom figure represent an opening 76 left by a circular disk of anterior stromal tissue that has been excised (e.g. with a trephine). Figures 11C and 11D show that the optic 72 may fit into opening 76. Figures 11E and 11F show that the optic 72 has been attached to the two halves of the rim 70 and 74 to complete assembly of the corneal implant. The individual pieces of the corneal implant may be attached to each other by interlocking fittings (not shown), by glue, or any other appropriate mechanical or chemical method of fixation. In this embodiment of the invention the corneal implant is shown as a three piece prosthesis that replaces part of the cornea. However, it is to be understood that the invention includes any corneal implant that can be assembled as two or more pieces within a corneal pocket.

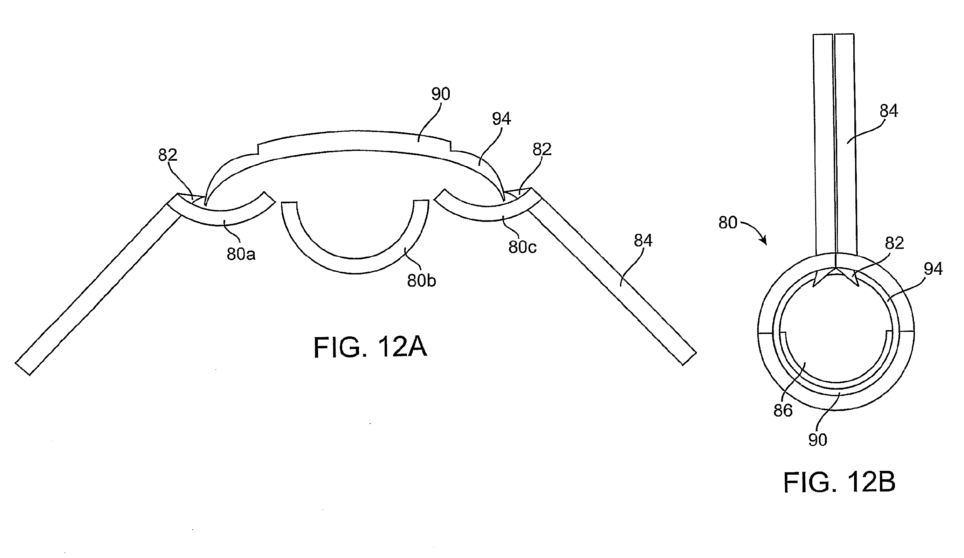

Figs. 12A-12B are end views of the back of a deformation chamber 86 on a hollow member 80 which show how the presence of a protrusion 82 within the deformation chamber can help to maintain the orientation of a corneal implant 90 as it is pushed in an axial direction. Deformation chamber 86 includes three hinged sections 80a, 80b, and 80c which make up a hollow member which opens in order to receive corneal implant 90. At the lateral aspects of deformation area 80 are two protrusions 82, which help to hold the rim 94 of corneal implant 90 in place. Fig. 12B shows how sections 80a, 80b, and 80c can be closed by putting together the wings 84 (which together form an axial pusher or implant mover) to create hollow member 80 and deformation chamber 86. Corneal implant 90 is now securely fixated within the hollow deformation chamber 86 by the protrusions 82 and can be manipulated by implant mover 84. The corneal implant 90 can then be moved axially along hollow member 80 by an axial pusher or other implant mover(not shown) without inadvertent rotation of the corneal implant.

Please note at least some portion of the corneal implant could be colored in any of the embodiments of the invention to enhance the aesthetic appearance of the eye or to decrease the amount of light exposure to the eye (e.g. for treatment of aniridia).

While the above is a complete description of the preferred embodiments of the invention, various alternatives, modifications, and equivalents may be used. Therefore, the above description should not be taken as limiting the scope of the invention which is defined by the appended claims.

### THE DISCLOSURE OF THIS APPLICATION ALSO INCLUDES THE FOLLOWING NUMBERED CLAUSES

1. A method for delivering a corneal implant to a cornea, said method comprising:
   constraining the implant to a small width configuration; and advancing the constrained implant laterally into a pocket which has been previously formed in the cornea, wherein the constrained implant assumes an unconstrained configuration within the pocket.
2. A method as in clause 1, wherein the implant has been constrained within a hollow member further comprising positioning the hollow member adjacent the pocket.
3. A method as in clause 2, wherein the implant has an unconstrained width and the hollow member is positioned through an opening to the pocket, wherein the opening has a width which is no greater than one-half the width of the unconstrained implant.
4. A method as in clauses 2, wherein constraining comprises placing the implant in its unconstrained configuration in a chamber coupled to the hollow member, rolling or folding the implant to constrain the implant within the chamber, and passing the constrained implant into a hollow passage in the hollow member.
5. A method as in clause 2, wherein advancing comprises pushing against the constrained implant to advance the implant out a distal end of the hollow member.
6. A system for delivering corneal implants, said system comprising: a hollow member having a proximal end and a distal end configured for insertion into a pocket within a cornea and having an axial hollow passage; and an axial pusher disposed in the hollow axial passage of the hollow member to engage and axially advance a constrained corneal implant through the hollow passage.
7. A system as in clause 6, further comprising a corneal implant constrained within the hollow passage on a distal side of the axial pusher.
8. A system as in clause 6, wherein the axial pusher is tapered in a distal direction.
9. A system as in clause 8, wherein the axial pusher is deformable so that it will reduce in diameter as it is distally advanced through the tapered hollow passage.
10. A system as in clause 6, further comprising an implant deformation chamber coupled to the hollow member.
11. A corneal implant comprising a protruding center optic at least partially surrounded by a rim.
12. A corneal implant as in clause 11, wherein center optic extends anteriorly from the rim.
13. A corneal implant as in clause 11, wherein the center optic extends posteriorly from the implant.
14. A corneal implant as in clause 11, wherein the implant comprises of a material selected from siloxane polymers, acrylic polymers, and collagen polymers.
15. A corneal implant as in clause 11, wherein the implant comprises a copolymer of a polymethacrylic acid and hydroxyethyl methacrylate (PHEMA/MAA).
16. An implant as in clause 11, wherein the implant is bonded with a material selected from the group consisting of fibronectin, laminin, substance P, insulin-like growth factor-1, or peptide sequences such as fibronectin adhesion-promoting peptide (FAP).
17. A corneal implant as in clause 11 in which at least a portion is colored.
18. A corneal implant as in clause 11 in which at least a portion of the implant is a lens.
19. A corneal lens implant as in clause 18, wherein the lens is of a type selected from the group consisting of monofocal, multifocal, Fresnel, diffractive, prismatic, and electromagnetic wave adjustable.
20. A corneal implant as in clause 11 in which the corneal implant contains an ultraviolet filter comprising a benzophenone.
21. A corneal implant as in clause 11, wherein at least a portion of the implant has holes or is porous.
22. A corneal implant comprising at least two pieces that can be assembled within a corneal pocket to form a functioning corneal implant.
23. A corneal implant as in clause 22, wherein the implant is composed of a material selected from siloxane polymers, acrylic polymers, and collagen polymers.
24. A corneal implant as in clause 22, wherein the implant comprises a copolymer of a polymethacrylic acid and hydroxyethyl methacrylate (PHEM A/M AA).
25. An implant as in clause 22, wherein the implant is bonded with a material selected from the group consisting of fibronectin, laminin, substance P, insulin-like growth factor- 1, or peptide sequences such as fibronectin adhesion-promoting peptide (FAP).
26. A corneal implant as in clause 22 in which at least a portion is colored.
27. A corneal implant as in clause 22 in which at least a portion of the implant is a lens.
28. A corneal lens implant as in clause 27, wherein the lens is one or more of the following types: monofocal, multifocal, Fresnel, diffractive, prismatic, and electromagnetic wave adjustable.
29. A corneal implant as in clause 22 in which the corneal implant contains an ultraviolet filter comprising a benzophenone.
30. A corneal implant as in clause 22, wherein at least a portion of the implant has holes or is porous.
31. A method for implanting a corneal implant in a cornea comprising: introducing a first portion of the prosthesis into a corneal pocket; introducing at least a second portion of the prosthesis into the corneal pocket; and assembling said first and at least a second portion of the implant to form a functional corneal implant.

## Claims

1. A corneal implant (50; 60), wherein the corneal implant (50; 60) is reversibly deformable, the deformed implant being capable of implantation in a corneal pocket through a corneal incision which is smaller than the width of the undeformed implant.

2. A corneal implant as in claim 1, wherein the deformed implant is capable of passage through a corneal incision which is equal to or less than half the width of the undeformed implant.

3. A corneal implant as in any of the preceding claims, wherein the implant (50; 60) comprises of a material selected from siloxane polymers, acrylic polymers, collagen polymers and a copolymer of a polymethacrylic acid and hydroxyethyl methacrylate, PHEMA/MAA.

4. An implant as in any of the preceding claims, wherein the implant (50; 60) is bonded with a material selected from the group consisting of fibronectin, laminin, substance P, insulin-like growth factor-1, or peptide sequences such as fibronectin adhesion-promoting peptide, FAP.

5. A corneal implant as in any of the preceding claims, in which at least a portion is colored.

6. A corneal implant as in any of the preceding claims, in which at least a portion of the implant (50; 60) is a lens.

7. A corneal lens implant as in claim 6, wherein the lens is of a type selected from the group consisting of monofocal, multifocal, Fresnel, diffractive, prismatic, and electromagnetic wave adjustable.

8. A corneal implant (50; 60) as in any of the preceding claims in which the corneal implant contains an ultraviolet filter comprising a benzophenone.

9. A corneal implant as in any of the preceding claims wherein the corneal implant comprises a miniature camera.

10. A corneal implant as in any of claims 1 to 8 wherein the corneal implant comprises a glucose monitor.

11. A corneal implant as in any of claims 1 to 8 wherein the corneal implant comprises a device for treating refractive error.

12. A corneal implant as in any of the preceding claims wherein at least a portion of the implant has holes or is porous.

13. A corneal implant as in any of the preceding claims wherein the corneal implant is deformable by rolling.

14. A corneal implant as in any of the preceding claims wherein the corneal implant is arranged to unfurl after implantation into the cornea.
